# EUROPEAN PATENT SPECIFICATION

(11) **EP 0 373 000 B1**
(45) Date of publication and mention of the grant of the patent: **25.02.1998**
(21) Application number: 89312855.3
(22) Date of filing: 08.12.1989
(51) Int. Cl.: G01R 33/28

(54) **Magnetic resonance apparatus**
Apparat mit magnetischer Resonanz
Appareil de résonance magnétique

(30) Priority: 09.12.1988 GB 8828810
(43) Date of publication of application: 13.06.1990
(73) Proprietor: PICKER INTERNATIONAL LIMITED, Wembley Middlesex HA9 7PR (GB)
(72) Inventor: Dickinson, Robert Julian, Wealdstone Middlesex (GB); Randell, Christopher Paul, Uxbridge Middlesex (GB)
(74) Representative: McGowan, Nigel George

(56) References cited:
- EP-A- 161 782
- EP-A- 245 755
- EP-A- 0 187 389
- EP-A- 0 314 262
- DE-A- 3 639 140
- JP-A- 026 052
- US-A- 4 681 308
- US-A- 4 771 785
- PATENT ABSTRACTS OF JAPAN, vol. 10, no. 298 (P-505)[2354], 9th October 1986; & JP-A-61 114 148 (SUMITOMO SPECIAL METALS CO., LTD) 31-05-1986

## Description

This invention relates to magnetic resonance (MR) apparatus.

MR apparatus includes, inter alia, a magnet system for producing a homogeneous static magnetic field of high strength in which the object to be examined, e.g. imaged, is positioned during examination, and which serves to define an equilibrium axis of magnetic alignment of nuclei within the object.

One particular application of MR apparatus is the examination of patients for medical diagnostic purposes. Such apparatus is often designed so as to be capable of examining any desired part of the whole body of a patient. The magnet system of such a so-called "whole body" MR apparatus is necessarily quite bulky and where, as is normally the case, the patient is inserted into the apparatus whilst lying horizontally in the direction of the head-to-toe axis of the patient, the MR apparatus together with the associated patient handling equipment requires a large floor area to accommodate it, i.e. has a large so-called "footprint".

JP-A-62-026052 discloses a patient loading MR apparatus for use with a C-shaped magnet. A patient support is cantilevered on a base. However, the support and the base impede access to the patient in the event that medical intervention becomes necessary. In particular, the cantilevered support extends outwardly away from the patient. Similarly, the base obstructs access to the patient, especially when the patient is positioned partially or fully within the gap.

According to the present invention there is provided a whole body MR apparatus including a magnet system including a magnetic core arrangement which is shaped so as to provide in a gap between surfaces of a pair of opposed parts of the core arrangement joined by a yoke a static magnetic field for defining an equilibrium axis of magnetic alignment of nuclei within a patient under examination; and a patient handling equipment including a movable patient support member of elongated form adapted to support a patient for examination, said patient support member being capable of translational movement in a plane transverse to the direction of the magnetic field in said gap in a direction transverse to the longitudinal axis of said support member, the extent of said movement being sufficient to allow a patient on said support member to be moved between a position within said gap and a position substantially wholly outside of said gap so as to avoid interference between the patient and the magnetic core arrangement as the patient is placed on and removed from the support member, characterised in that said patient handling equipment is mounted on said magnetic core arrangement.

Typically said plane is orthogonal to said magnetic field direction.

Preferably said support member is further capable of translational movement in said plane in a second direction at an angle to the first-mentioned direction of translational movement at least when said support member is at a position such that said patient is within said gap.

Preferably said first-mentioned and second directions are respectively orthogonal to and parallel to said longitudinal axis.

In one particular embodiment of the invention said support member is capable only of translational movement in said plane.

In another particular embodiment of the invention said support member is also capable of rotational movement about axes normal to said plane.

The support member is suitably adapted to support a patient whilst lying horizontally with the head-to-toe axis of the patient parallel to said longitudinal axis of the support member.

It will be understood that magnet systems for MR apparatus which provide a magnetic field for defining an equilibrium axis in a gap between surfaces of opposed parts of a magnetic core arrangement are known per se, see for example EP-A-161782.

An MR apparatus with a three-axis patient positioning assembly is described in US-A-4771785. However for moving a patient into and out of the magnet system the assembly moves the patient in the direction of the head-to-toe axis of the patient.

In DE-A-3639140 there is described an MR apparatus which includes a patient support member which can be moved in a direction transverse to the magnet field direction and the longitudinal axis of the support member, but the movement is such only as to enable examination of different regions of a patient.

Several MR apparatus in accordance with the invention will now be described, by way of example, with reference to accompanying drawings in which:-
Figure 1 is a schematic diagram showing the general arrangement of all the apparatuses to be described;
Figure 2 is a diagram illustrating a magnetic core arrangement of a magnet system of a first apparatus according to the invention;
Figures 3, 4 and 5 are respectively diagrammatic plan, side and end views of the magnetic core arrangement and a patient handling equipment of the first apparatus;
Figure 6 is a diagram illustrating an alternative form for the magnetic core arrangement of the first apparatus;
Figure 7 is a diagram illustrating a magnetic core arrangement of a second apparatus in accordance with the invention;
Figure 8 is a diagrammatic plan view of the magnetic core arrangement and a patient handling system of the second apparatus; and
Figures 9 and 10 are respectively diagrammatic plan and side views of a magnetic core arrangement and patient handling equipment of a third apparatus according to the invention.

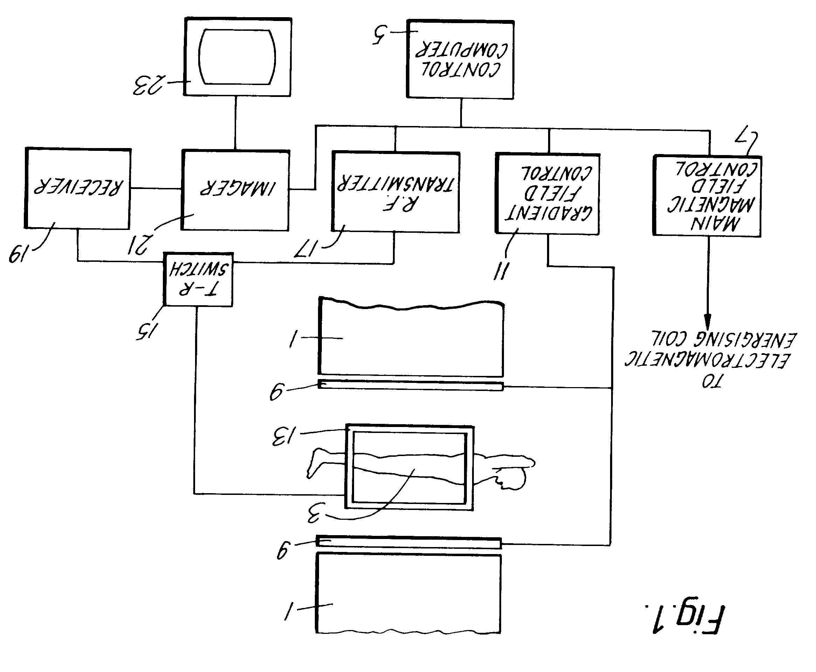

Referring to Figure 1, the apparatuses, which are diagnostic patient imaging apparatuses, include an electromagnet which produces a strong uniform static main magnetic field Bo across a gap between two pole pieces 1 of a magnetic core arrangement of the electromagnet, the pole pieces 1 being joined by a yoke (not shown in Figure 1) carrying an energising coil (not shown). In use of the apparatus a patient 3 to be imaged is placed in the gap between the pole pieces lying horizontally on a support member of a patient handling equipment (not shown) so that the head-to-toe axis of the patient 3 is orthogonal to the direction of the magnetic field in the gap. The strength of the field in the gap between the pole pieces 1, and hence in the patient's body, is controlled by a computer 5 via a main magnet control means 7 which controls the supply of energising current to the electromagnet energising coil.

The apparatus further includes a gradient coil system 9 whereby a gradient may be imposed on the static magnetic field in the gap between the pole pieces 1 in any one or more of three orthogonal directions. The coil system 9 is energised by a gradient field control means 11 under control of the computer 5.

The apparatus further includes an r.f. antenna system 13.

The antenna system 13 is selectively connectable by way of a transmit-receive switch 15 either to an r.f. transmitter 17 or a receiver 19. The transmitter 17 is operated under control of the computer 5 to apply r.f. field pulses to the patient's body 3 for excitation of magnetic resonance in the patient's body 3. R.f. signals resulting from magnetic resonance excited in the body are sensed by the antenna system 13 and passed via the receiver 19 to an imager 21 which under control of the computer 5 processes the signals to produce signals representing an image of the body 3. These signals are, in turn, passed to a display device 23 to provide a visual display of the image.

In operation of the apparatus the field provided by the electromagnet defines an equilibrium axis of magnetic alignment in the body 3.

To obtain an image of a selected region, e.g. a cross-sectional slice of the patient's body 3, an r.f. field pulse is first applied to the body 3 by means of the r.f. transmitter 17 and antenna system 13 to excite magnetic resonance in the selected region. To this end the antenna system 13 produces a field B1 in a direction orthogonal to the static field direction so as to tip the spins of nuclei in the selected region from the direction of the static field into a plane orthogonal to the static field direction. To restrict excitation to the selected region the r.f. field pulse is applied in conjunction with magnetic field gradients imposed by the coil system 9, the frequency of the r.f. field being chosen in conjunction with the magnitudes and directions of the imposed gradients so that the Larmor frequency of chosen protons in the body, e.g. hydrogen protons, is equal to the r.f. field frequency only in the selected region.

The r.f. signals resulting from excitation are then spatially encoded by application of one or more further gradient magnetic fields in known manner, detected by the antenna system 13 and processed to produce an image.

Normally a number of excitation and signal detection sequences are required to produce sufficient data to produce a satisfactory image.

Instead of using a T-R switch, the antenna system 13 may comprise separate transmitting and receiving coil arrangements respectively connected with transmitter 17 and receiver 19.

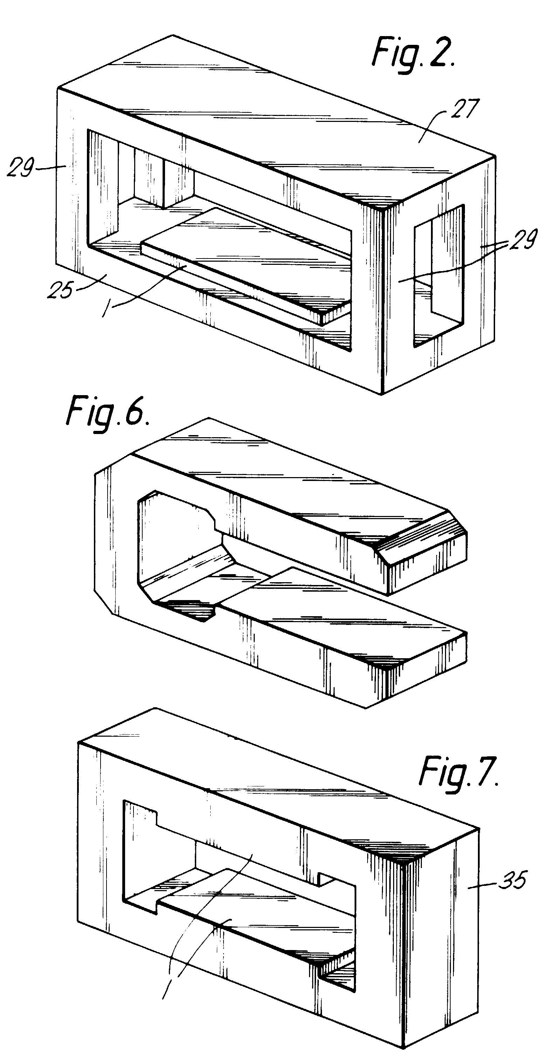

In one embodiment of the apparatus of Figure 1 in accordance with the present invention, the electromagnet is of the so-called four-poster bed type, i.e. the magnetic core arrangement of the electromagnet Is similar in shape to a four-poster bed, as shown in Figure 2. Thus the yoke joining the pole pieces 1 comprises horizontal spaced apart planar rectangular base and roof portions 25 and 27 joined by vertical posts 29, one at each corner, with the pole pieces 1 extending towards one another from the centre of the base and roof portions 25 and 27 respectively.

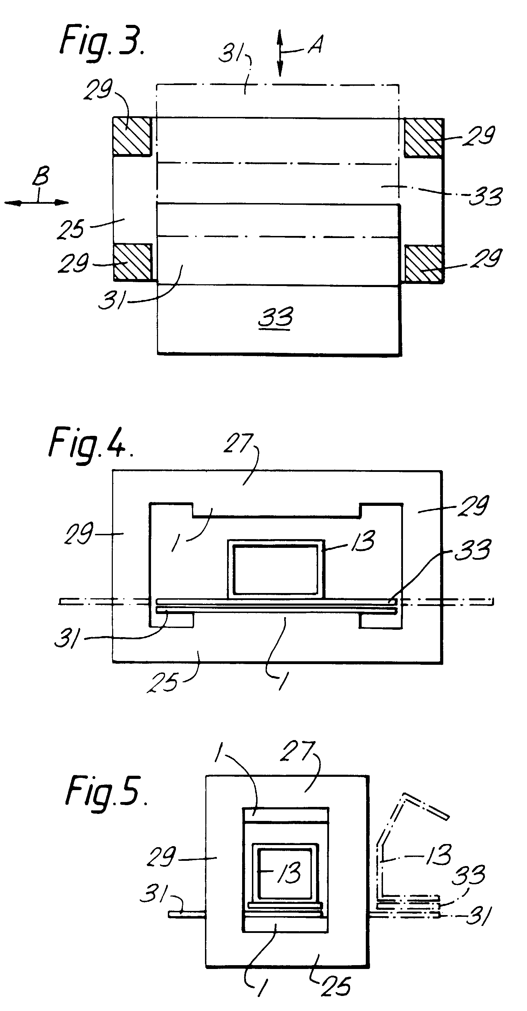

Referring to Figures 3, 4 and 5, the patient handling equipment of this embodiment comprises a lower horizontal rectangular planar table portion 31 mounted with its longer axis parallel to the longer horizontal axis of the magnetic core arrangement on rails or rollers (not shown), just above the face of the lower pole piece 1, so as to be slidable in a horizontal plane in a direction orthogonal to the longer axis of the core arrangement. On its upper surface adjacent a longer side the table portion 31 carries an elongated rectangular patient support member or platter 33 arranged to be slidable on the table portion 31, e.g. on rails or rollers, in the direction of its longer axis, which axis is parallel to the longer axis of the table portion 31.

The patient platter 33 is thus capable of translational movement in two directions in a plane orthogonal to the direction of the magnetic field in the gap between the pole pieces 1, the two directions being respectively orthogonal and parallel to the longer axis of the platter 33, as indicated by arrows A and B in Figure 3.

In use of the apparatus the table portion 31 is slid out from the core arrangement from the side at which the platter 33 is mounted so as to move the platter 33 in the direction of arrow A orthogonal to its longer axis to a position in which it is wholly outside the core arrangement, as shown in full lines in Figure 3. The patient is then placed on the platter 33 and the table portion 31 slid back into the core arrangement to bring the platter 33, and hence the patient's body, to a position where it extends centrally through the gap between the pole pieces, as shown in chain-dotted lines in Figure 3. The platter 33 can then be slid along the table portion 31 in the direction of arrow B, i.e. the direction of its longer axis, to bring the particular section of the patient's body it is desired to image to a position centrally between the pole pieces 1, if necessary passing between the closer spaced pairs of the posts 29, as shown in chain-dotted lines in Figure 4.

The r.f. antenna system 13 is suitably carried on the patient platter 33 and where, as is normally the case, it is required to surround the patient's body, it may be of a hinged construction to allow side access to the platter 33, as illustrated in dotted lines in Figure 5.

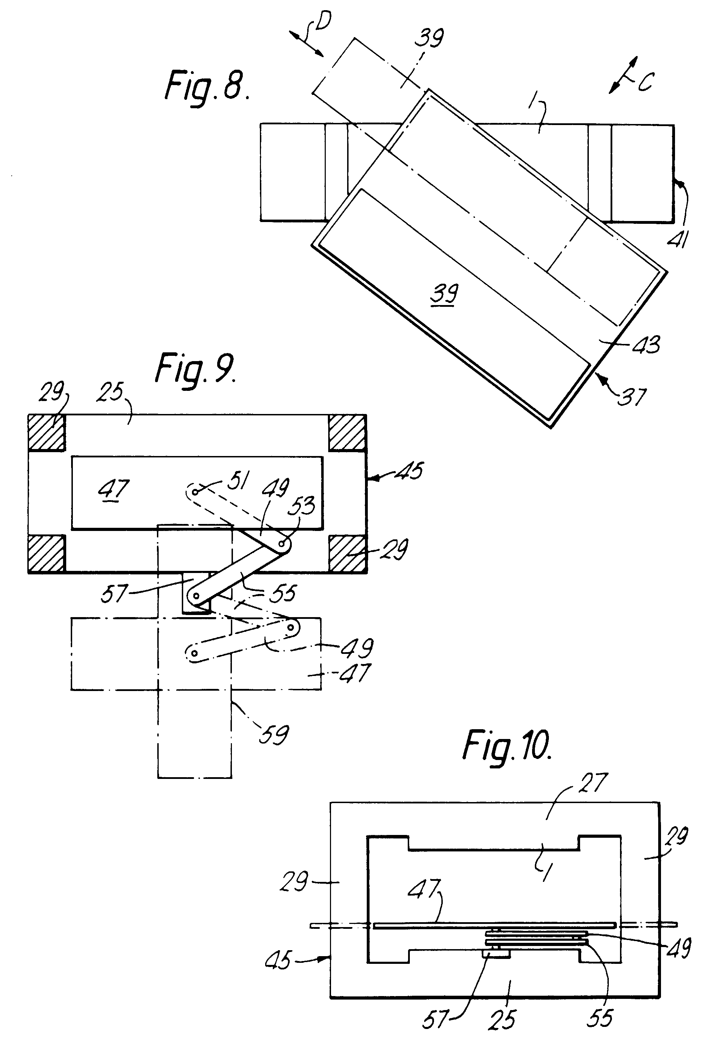

It will be appreciated that the form of patient handling equipment illustrated in Figures 3, 4 and 5 is also suitable for use with magnetic core arrangement of C-shape, as illustrated in Figure 6.

Where the magnetic core arrangement is of so-called H-form, as illustrated in Figure 7, i.e the pole pieces 1 are joined by a yoke 35 of planar rectangular form with the pole pieces 1 positioned centrally on opposite limbs of the yoke 35, a patient handling equipment 37 of similar form to that illustrated in Figures 3 to 5 may be used. However, in this case the longer axis of the patient support platter 39 is at an acute angle to the longer axis of the core arrangement 41, as illustrated in Figure 8, and the patient platter 39 is slidable in two orthogonal directions, indicated by arrows C and D, on a lower table 43 which is fixed with respect to the magnet core arrangement 41. The platter 39 when slid into a position adjacent the gap between the pole pieces 1 by movement in direction C orthogonal to its longer axis is then able to be slid in direction D so as to pass diagonally through the core arrangement 41 via the gap between the pole pieces 1.

In further embodiments of the invention, suitable for use with magnetic core arrangements of any of the above-described forms, the patient-handling equipment comprises a patient support member arranged for rotational motion as well as translational motion.

One such embodiment is illustrated in Figures 9 and 10 wherein a four-poster bed magnetic core arrangement 45 is shown by way of example. In the embodiment of Figures 9 and 10 the patient handling equipment comprises a horizontal planar rectangular patient support member 47 which at a central position on its underside is mounted on one end of a horizontal arm 49 for pivotal movement about a vertical axis 51. At its other end the arm 49 is, in turn, mounted for pivotal motion about a vertical axis 53 at one end of a second horizontal arm 55 whose other end is similarly pivotally connected to a flange member 57 fixed with respect to the core arrangement 45.

The support member 47 is thus capable of translational motion, with its longer axis parallel to the longer horizontal axis of the core arrangement 45, in directions orthogonal and parallel to its own longer axis, as indicated by chain-dotted lines in Figures 9 and 10, in exactly the same manner as the platter 33 of Figures 3 to 5. In addition, the support member 47 is capable of rotational movement in a horizontal plane at any position reached by the translational movement.

Such an arrangement exhibits several advantages. For example, if space permits, when the patient is removed from the core arrangement, the member 47 may be rotated through 90° to the position indicated by dotted line 59 in Figure 9 to permit access to both sides of a patient. In addition, positioning of the patient's head-to-toe axis other than parallel to the longer axis of the core arrangement 45 is possible, and the direction of the patient's head-to-toe axis may be reversed without removing the patient from the support member 47. The latter feature may be of particular benefit if the available space severely limits movement of the support member 47 in the direction of the longer axis of the core arrangement 45.

It will be appreciated that the apparatus described above by way of example with reference to Figures 3 to 5 and Figure 8 may easily be modified to permit rotational movement of the support members 33 and 39.

## Claims

1. A whole body magnetic resonance apparatus including a magnet system including a magnetic core arrangement (1, 25, 27, 29 or 41 or 45) which is shaped so as to provide in a gap between surfaces of a pair of opposed parts (1) of the core arrangement (1, 25, 27, 29 or 41 or 45) joined by a yoke (25, 27, 29 or 35) a static magnetic field for defining an equilibrium axis of magnetic alignment of nuclei within a patient (3) under examination; and a patient handling equipment (31, 33 or 37 or 47 to 55) including a movable patient support member (33, 39 or 47) of elongated form adapted to support a patient (3) for examination, said patient support member (33, 39 or 47) being capable of translational movement in a plane transverse to the direction of the magnetic field in said gap in a direction (A or C) transverse to the longitudinal axis of said support member (33, 39 or 47), the extent of said movement being sufficient to allow a patient on said support member to be moved between a position within said gap and a position substantially wholly outside of said gap so as to avoid interference between the patient (3) and the magnetic core arrangement (1, 25, 27, 29 or 41 or 45) as the patient (3) is placed on and removed from the support member (33, 39 or 37), characterised in that said patient handling equipment (31, 33 or 37 or 47 to 55) is mounted on said magnetic core arrangement (1, 25, 27, 29 or 41 or 45).

2. An apparatus according to Claim 1 wherein said plane is orthogonal to said magnetic field direction.

3. An apparatus according to Claim 1 or Claim 2 wherein said support member (33, 39 or 47) is further capable of translational movement in said plane in a second direction (B or D) at an angle to the first-mentioned direction (A or C) of translational movement at least when said support member (33, 39 or 47) is at a position such that said patient (3) is within said gap.

4. An apparatus according to Claim 3 wherein said support member (33) is mounted slidably on an underlying member (31) for translational movement with respect to said underlying member (31) in said second direction (B), and said underlying member (31) is slidably mounted with respect to said core arrangement (1, 25, 27, 29) for said translational movement of said support member (33) in said first-mentioned direction (A).

5. An apparatus according to Claim 3 wherein said support member (39) is mounted slidably on an underlying member (43) for translational movement with respect to said underlying member (43) both in said first-mentioned direction (C) and in said second direction (D).

6. An apparatus according to Claim 3, 4 or 5 wherein said first-mentioned and second directions (A, B or C, D) are respectively orthogonal to and parallel to said longitudinal axis.

7. An apparatus according to Claim 6 wherein said core arrangement (1, 25, 27, 29 or 45) is of rectangular cross-section in planes orthogonal to the direction of said magnetic field and the longer sides of said cross-section are substantially orthogonal to said first-mentioned direction (A).

8. An apparatus according to Claim 7 when dependent on Claim 4 wherein said core arrangement (1, 25, 27, 29) is a four-poster bed core arrangement; said underlying member (31) is arranged to pass between the pairs of posts (29) of the core arrangement (1, 25, 27, 29) at opposite ends of the longer sides of the core arrangement (1, 25, 27, 29) when slid in said first-mentioned direction (A); and said support member (33) is arranged to pass between the pairs of posts (29) of the core arrangement (1, 25, 27, 29) at opposite ends of the shorter sides of the core arrangement (1, 25, 27, 29) when slid in said second direction (B).

9. An apparatus according to Claim 6 when dependent on Claim 5 wherein said opposed parts (1) are joined by a yoke portion (35) of the core arrangement (41) of planar rectangular form with said surfaces respectively positioned centrally along a pair of opposite limbs of the yoke portion (35); and said support member (39) is disposed with its longitudinal axis at an acute angle to the longer axis of said core arrangement (41) so as to be capable of passing diagonally through said core arrangement (41) via said gap when undergoing said translational movement in said second direction (D) when in at least one position along said first-mentioned direction (C).

10. An apparatus according to any one of the preceding claims wherein said support member (33 or 39) is capable only of translational movement in said plane.

11. An apparatus according to any one of Claims 1 to 9 wherein said support member (47) is also capable of rotational movement about axes normal to said plane.

12. An apparatus according to Claim 11 wherein said support member (47) is pivotally mounted for rotation in said plane at one end of a first arm (49) whose other end is pivotally mounted to one end of a second arm (55) which at its other end is pivotally mounted for rotation about an axis fixed with respect to said core arrangement (45).

13. An apparatus according to any one of the preceding claims wherein said support member (33, 39 or 47) is adapted to support a patient (3) whilst lying horizontally with the head-to-toe axis of the patient (3) parallel to said longitudinal axis of the support member (33, 39 or 47).

## Patentansprüche

1. Ein Ganzkörper-Magnet-Resonanz-Apparat, enthaltend ein Magnetsystem enthaltend eine Magnetkernanordnung (1, 25, 27, 29 oder 41 oder 45), die so geformt ist, daß sie in einem Zwischenraum zwischen den Flächen eines Paares von gegenüberliegenden Teilen (1) der Kernanordnung (1, 25, 27, 29 oder 41 oder 45), die durch ein Joch (25, 27, 29 oder 35) verbunden sind, ein statisches Magnetfeld zur Festlegung einer Gleichgewichtsachse der magnetischen Ausrichtung der Kerne in einem untersuchten Patienten (3) liefert; und eine Einrichtung zur Handhabung des Patienten (31, 33, oder 37 oder 47 bis 55) enthaltend ein bewegliches Patiententragelement (33, 39 oder 47) von länglicher Form, dazu angepaßt den zu untersuchenden Patienten (3) zu tragen, wobei das genannte Patiententragelement (33, 39, oder 47) Translationsbewegungen in einer Ebene transversal zu der Richtung des Magnetfeldes im genannten Zwischenraum in einer Richtung (A oder C) transversal zu der Längsachse des genannten Tragelements (33, 39 oder 47) zuläßt, wobei das Ausmaß der genannten Bewegung ausreicht, um zuzulassen, daß der Patient auf dem genannten Tragelement zwischen einer Position innerhalb des genannten Zwischenraums und einer Position im wesentlichen völlig außerhalb des genannten Zwischenraums bewegt werden kann, so daß eine Kollision zwischen dem Patienten (3) und der Magnetkernanordung (1, 25, 27, 29 oder 41 oder 45) vermieden wird, wenn der Patient (3) auf das Tragelement (33, 39 oder 47) gebracht oder von ihm entfernt wird, dadurch gekennzeichnet, daß die genannte Einrichtung (31, 33 oder 37 oder 47 bis 55) zur Handhabung des Patienten auf der genannten Magnetkernanordnung (1, 25, 27, 29 oder 41 oder 45) montiert ist.

2. Ein Apparat gemäß Anspruch 1, bei dem die genannte Ebene senkrecht zu der genannten Magnetfeldrichtung ist.

3. Ein Apparat gemäß einem der Ansprüche 1 oder 2, bei dem das genannte Tragelement (33, 39 oder 47) weiterhin zu Translationsbewegungen in der genannten Ebene in einer zweiten Richtung (B oder D) in einem Winkel zu der erstgenannten Richtung (A oder C) von Translationsbewegungen in der Lage ist, zumindest wenn das genannte Tragelement (33, 39 oder 47) in einer solchen Position ist, daß der genannte Patient (3) innerhalb des genannten Zwischenraums ist.

4. Ein Apparat gemäß Anspruch 3, bei dem das genannte Tragelement (33) auf einem darunterliegenden Bauelement (31) verschiebbar für Translationsbewegungen in Bezug auf das genannte darunter liegende Bauelement (31) in der genannten zweiten Richtung (B) befestigt ist, und bei dem das darunterliegende Bauelement (31) verschiebbar in Bezug auf die genannte Kernanordnung (1, 25, 27, 29) für die genannte Translationsbewegung des genannten Tragelements (33) in der genannten erstgenannten Richtung (A) befestigt ist.

5. Ein Apparat gemäß Anspruch 3, bei dem das genannte Tragelement (39) auf einem darunterliegenden Bauelement (43) verschiebbar für Translationsbewegungen in Bezug auf das genannte darunterliegende Bauelement (43) in beide Richtungen, die erstgenannte Richtung (C) und die genannte zweite Richtung (D), befestigt ist.

6. Ein Apparat gemäß einem der Ansprüche 3, 4 oder 5, bei dem die erstgenannte und die zweite Richtung (A, B oder C, D) senkrecht bzw. parallel zu der genannten Längsachse sind.

7. Ein Apparat gemäß Anspruch 6, bei dem die genannte Kernanordnung (1, 25, 27, 29 oder 45) rechteckigen Querschnitt in den Ebenen senkrecht zu der Richtung des genannten Magnetfeldes hat und bei dem die längeren Seiten des genannten Querschnitts im wesentlichen senkrecht zu der genannten erstgenannten Richtung (A) sind.

8. Ein Apparat gemäß Anspruch 7, wenn dieser von Anspruch 4 abhängt, bei dem die genannte Kernanordnung (1, 25,27, 29) eine "Himmelbett"-Kernanordnung ist; bei dem das genannte darunterliegende Bauelement (31) so angeordnet ist, daß es zwischen dem Paar von Pfosten (29) der Kernanordnung (1, 25, 27, 29) an gegenüberliegenden Enden der längeren Seiten der Kernanordnung (1, 25, 27, 29) durchgeht, wenn es in der zuerst erwähnten Richtung (A) geschoben wird; und bei dem das genannte Tragelement (33) so angeordnet ist, daß es zwischen dem Paar von Pfosten (29) der Kernanordnung (1, 25, 27, 29) an gegenüberliegenden Enden der kürzeren Seiten der Kernanordnung (1, 25, 27, 29) durchgeht, wenn es in der genannten zweiten Richtung (B) verschoben wird.

9. Ein Apparat gemäß Anspruch 6, wenn dieser von Anspruch 5 abhängt, bei dem die gegenüberliegenden Teile (1) verbunden sind durch ein Jochstück (35) der Kernanordnung (41) von ebener, rechteckiger Form, wobei die genannten Flächen zentral entlang eines Paares von gegenüberliegenden Schenkeln des Jochstückes (35) angeordnet sind; und bei dem das genannte Tragelement (39) mit seiner Längsachse in einem spitzen Winkel zu der längeren Achse der genannten Kernanordnung (41) angeordnet ist, so daß es diagonal über den genannten Zwischenraum durch die genannte Kernanordnung (41) gehen kann, wenn es die genannte Translationsbewegung in der genannten zweiten Richtung (D) ausführt, wenn es an mindestens einer Stelle entlang der zuerst erwähnten Richtung (C) ist.

10. Ein Apparat gemäß einem der vorhergehenden Ansprüche, bei dem das genannte Tragelement (33 oder 39) nur zu Translationsbewegungen in der genannten Ebene in der Lage ist.

11. Ein Apparat gemäß einem der Ansprüche 1 bis 9, bei dem das genannte Tragelement (47) auch zu Rotationsbewegungen um eine Achse senkrecht zu der genannten Ebene in der Lage ist.

12. Ein Apparat gemäß Anspruch 11, bei dem das Tragelement (47) schwenkbar für Rotationen in der genannten Ebene an einem Ende eines ersten Arms (49) befestigt ist, dessen anderes Ende schwenkbar an einem Ende eines zweiten Arms (55) befestigt ist, der an seinem anderen Ende schwenkbar für Rotationen um eine in Bezug auf die genannte Kernanordnung (45) feststehende Achse befestigt ist.

13. Ein Apparat gemäß einem der vorhergehenden Ansprüche, bei dem das genannte Tragelement (33, 39 oder 47) darauf angepaßt ist, den Patienten (3) zu tragen, während er horizontal mit der Körperlängsachse des Patienten (3) parallel zu der Längsachse des Tragelements liegt (33, 39 oder 47).

## Revendications

1. Appareil de résonance magnétique du corps entier, comprenant un système à aimants qui comporte un ensemble à noyau magnétique (1, 25, 27, 29 ou 41 ou 45) qui a une configuration telle que, dans un entrefer compris entre les surfaces d'une paire de parties opposées (1) de l'ensemble à noyau (1, 25, 27, 29 ou 41 ou 45) raccordé par une culasse (25, 27, 29 ou 35), il forme un champ magnétique statique destiné à déterminer un axe d'équilibre d'alignement magnétique des noyaux dans un patient (3) examiné, et un appareillage de déplacement de patient (31, 33 ou 37 ou 47 à 55) comprenant un organe mobile (33, 39 ou 47) de support de patient de forme allongée destiné à supporter un patient (3) qui doit être examiné, l'organe (33, 39 ou 47) de support du patient pouvant présenter un mouvement de translation dans un plan transversal à la direction du champ magnétique dans l'entrefer dans une direction (A ou C) transversale à l'axe longitudinal de l'organe de support (33, 39 ou 47), l'amplitude de ce déplacement étant suffisante pour permettre à un patient placé sur l'organe de support d'être déplacé entre une position dans l'entrefer et une position pratiquement à l'extérieur en totalité de l'entrefer afin que le contact entre le patient (3) et l'ensemble à noyau magnétique (1, 25, 27, 29 ou 41 ou 45) soit évité lorsque le patient (3) est placé sur l'organe de support (33, 39 ou 37) ou retiré de cet organe, caractérisé en ce que l'appareillage (31, 33 ou 37 ou 47 à 55) de déplacement du patient est monté sur l'ensemble à noyau magnétique (1, 25, 27, 29 ou 41 ou 45).

2. Appareil selon la revendication 1, dans lequel le plan est perpendiculaire à la direction du champ magnétique.

3. Appareil selon la revendication 1 ou 2, dans lequel l'organe de support (33, 39 ou 47) est en outre capable de présenter un mouvement de translation dans ledit plan dans une seconde direction (B ou D) suivant un angle avec la première direction précitée (A ou C) du mouvement de translation au moins lorsque l'organe de support (33, 39 ou 47) occupe une position telle que le patient (3) se trouve dans l'entrefer.

4. Appareil selon la revendication 3, dans lequel l'organe de support (33) est monté afin qu'il puisse coulisser sur un organe (31) placé au-dessous de lui et qu'il présente un mouvement de translation par rapport à l'organe placé au-dessous de lui (31) dans la seconde direction (B), et l'organe placé au-dessous de lui (31) est monté afin qu'il puisse coulisser par rapport à l'ensemble à noyau (1, 25, 27, 29) et que l'organe de support (33) puisse présenter le mouvement de translation dans la première direction précitée (A).

5. Appareil selon la revendication 3, dans lequel l'organe de support (39) est monté afin qu'il puisse coulisser sur un organe placé au-dessous de lui (43) et présente un mouvement de translation par rapport à l'organe placé au-dessous de lui (43) à la fois dans la première direction précitée (C) et dans la seconde direction (D).

6. Appareil selon la revendication 3, 4 ou 5, dans lequel la première et la seconde direction (A, B ou C, D) sont respectivement perpendiculaire et parallèle à l'axe longitudinal.

7. Appareil selon la revendication 6, dans lequel l'ensemble à noyau (1, 25, 27, 29 ou 45) a une section rectangulaire dans les plans perpendiculaires à la direction du champ magnétique, et les grands côtés de la section sont pratiquement perpendiculaires à la première direction précitée (A).

8. Appareil selon la revendication 7 lorsqu'elle dépend de la revendication 4, dans lequel l'ensemble à noyau (1, 25, 27, 29) est un ensemble à noyau du type à lit en baldaquin, l'organe placé au-dessous de lui (31) est destiné à passer entre les paires de montants (29) de l'ensemble à noyau (1, 25, 27, 29) aux extrémités opposées des grands côtés de l'ensemble à noyau (1, 25, 27, 29) lors du coulissement dans la première direction précitée (A), et l'organe de support (33) est destinée à passer entre les paires de montants (29) de l'ensemble à noyau (1, 25, 27, 29) aux extrémités opposées des petits côtés de cet ensemble (1, 25, 27, 29) lors du coulissement dans la seconde direction (B).

9. Appareil selon la revendication 6, lorsqu'elle dépend de la revendication 5, dans lequel les parties opposées (1) sont raccordées par une partie de culasse (35) de l'ensemble à noyau (41) de forme rectangulaire plane, les surfaces étant respectivement positionnées au centre le long d'une paire de branches opposées de la partie de culasse (35), et l'organe de support (39) est disposé avec son axe longitudinal qui fait un angle aigu avec le grand axe de l'ensemble à noyau (41) afin qu'il puisse passer en diagonale dans l'ensemble à noyau (41) dans l'entrefer lors du déplacement en translation dans la seconde direction (D) alors qu'il se trouve au moins dans une position dans la première direction précitée (C).

10. Appareil selon l'une quelconque des revendications précédentes, dans lequel l'organe de support (33 ou 39) peut simplement se déplacer en translation dans ledit plan.

11. Appareil selon l'une quelconque des revendications 1 à 9, dans lequel l'organe de support (47) peut aussi présenter un mouvement de rotation autour d'axes perpendiculaires audit plan.

12. Appareil selon la revendication 11, dans lequel l'organe de support (47) est monté de manière pivotante afin qu'il tourne dans ledit plan à une première extrémité d'un premier bras (49) dont l'autre extrémité est montée afin qu'elle puisse pivoter sur une première extrémité d'un second bras (55) qui, à son autre extrémité, est monté de manière pivotante afin qu'il puisse tourner autour d'un axe fixé par rapport à l'ensemble à noyau (45).

13. Appareil selon l'une quelconque des revendications précédentes, dans lequel l'organe de support (33, 39 ou 47) est destiné à supporter un patient (3) lorsqu'il est placé horizontalement, l'axe allant de la tête aux pieds du patient (3) étant parallèle à l'axe longitudinal de l'organe de support (33, 39 ou 47).
